(19) **Europäisches Patentamt**

**European Patent Office**

**Office européen des brevets**

(11) **EP 0 673 640 B1**

(12) **EUROPEAN PATENT SPECIFICATION**

(45) Date of publication and mention
of the grant of the patent:
**23.05.2001 Bulletin 2001/21**

(21) Application number: **93921088.6**

(22) Date of filing: **29.09.1993**

(51) Int Cl.⁷: **A61K 7/09**

(86) International application number:
**PCT/JP93/01393**

(87) International publication number:
**WO 95/08977 (06.04.1995 Gazette 1995/15)**

(54) **INTERMEDIATE PERMANENT WAVE TREATMENT**

ZWISCHENBEHANDLUNG FÜR DAUERWELLEN

TRAITEMENT INTERMEDIAIRE POUR LA PERMANENTE

(84) Designated Contracting States:
**DE**

(43) Date of publication of application:
**27.09.1995 Bulletin 1995/39**

(73) Proprietor: **Kao Corporation**
**Chuo-Ku Tokyo 103 (JP)**

(72) Inventors:
• **WATAYA, Kazuyo**
**Chiba-shi, Chiba 261 (JP)**
• **KOGA, Hiroyuki**
**Chiba 273 (JP)**
• **KURE, Naohisa**
**Tokyo 136 (JP)**

(74) Representative:
**Hansen, Bernd, Dr. Dipl.-Chem. et al**
**Hoffmann Eitle,**
**Patent- und Rechtsanwälte,**
**Arabellastrasse 4**
**81925 München (DE)**

(56) References cited:
**JP-A- 3 153 621**

• **MANUFACTURING CHEMIST , vol. 59, no. 4, April 1988, LONDON GB, pages 42-44, XP002014944 "Permanent waving"**
• **Deutsches Arzneibuch (German Pharmacopoeia) 9th edition p. 288, p. 290 Deutscher Apotheker Verlag, Stuttgart 1986**

## Description

The present invention relates to the use of an intermediate treatment composition for permanent wave, and more particularly of an intermediate treatment composition for permanent wave, which serves to cleave mixed disulfides formed in the hair by a treatment with a first-package permanent wave composition which is within a range of from acidity to weak alkalinity, thereby enhancing permanent wave-forming ability, and can prevent damage to the hair to be caused by permanent waving.

BACKGROUND ART

Permanent waving for giving the hair the desired waves is a method in which S-S bonds in the hair are subjected to reductive chain cleavage with a first-package permanent wave composition (hereinafter referred to as "the first composition") comprising, as a principal component, a reducing agent such as thioglycolic acid or cysteine, and then to oxidative chain closure with a second-package permanent wave composition (hereinafter referred to as "the second composition") comprising, as a principal component, an oxidizing agent such as a bromate, perborate or aqueous hydrogen peroxide.

However, the reduction of the hair by the first composition is generally conducted under alkaline conditions of pH 8-10. Therefore, such phenomena that proteins and/or lipids in the hair are dissolved out, the strength of the hair is lowered and the feel of the hair to the touch is deteriorated have been caused. Such hair has involved a problem that when the hair is groomed to fix in a desired hair style, smooth brushing or combing can not be performed so that a brush or comb may be caught by the hair, which causes separation of hair cuticle, split hairs and broken hairs, resulting in damage to the hair.

Therefore, various intermediate treatment compositions for permanent wave have heretofore been provided with a view toward preventing such damage to the hair by the permanent waving to improve a feel of the hair to the touch, and enhancing wave-forming ability. For example, the damage to the hair is prevented by adding a hydrolyzate of keratin as described in Japanese Patent Application Laid-Open No. 246509/1987, or by adding a hydrolyzate of a protein as described in GB 2 160 419 A. Further, in U.S. Patent No. 4,494,557, magnesium sulfate and a mixture of a hydrolyzate of a protein, oily substance, surfactant, inorganic salt and the like are used to enhance wave-forming ability and hence to keep waves longer.

However, the conventional intermediate treatment compositions for permanent wave can only suppress a feeling of damage to the hair and improve the feel of the hair to the touch, but neither essentially improve the damage to the hair, nor have satisfactory wave-forming ability.

On the other hand, low-damage permanent waving in which the pH of a first-package permanent wave composition has been adjusted within a range of from acidity to weak alkalinity for the purpose of suppressing the swelling of the hair and preventing proteins and amino acids from dissolving out has been conducted. However, in such a pH, mixed disulfides which are combination products of a cysteine residue in the hair with a mercapto compound are formed during the reductive cleavage of S-S bonds by a reducing agent such as thioglycolic acid, cysteine or glyceryl monothioglycolate, so that the subsequent oxidation by the second composition becomes incomplete. Therefore, such a treatment has involved a drawback that waving efficiency in the permanent waving becomes low, and the damage to the hair is also unavoidable.

It is thus an object of the present invention to provide an intermediate treatment composition for permanent wave, which can provide excellent wave-forming ability even in permanent waving making use of the first composition whose pH is within a range of from acidity to weak alkalinity, and prevent damage to the hair.

In view of the foregoing circumstances, the present inventors have carried out an extensive investigation. As a result, it has been found that carbonates and hydrogencarbonates are useful for the cleavage of mixed disulfides, and the use of an intermediate treatment composition for permanent wave with these salts incorporated therein permits both enhancement of wave-forming rate for the hair and prevention of damage to the hair, leading to completion of the present invention.

DISCLOSURE OF THE INVENTION

The present invention is directed to the use of an intermediate treatment composition for permanent wave, comprising at least one carbonate and/or at least one hydrogencarbonate.

The present invention is also related to a permanent-waving process, which comprises treating the hair with a first-package permanent wave composition, with an intermediate treatment composition for permanent wave including at least one carbonate and/or at least one hydrogencarbonate and then with a second-package permanent wave composition.

The intermediate treatment composition for permanent wave according to the present invention serves to cleave mixed disulfides formed in the hair by a treatment with a first-package permanent wave composition which is within a range of from acidity to weak alkalinity, thereby enhancing permanent wave-forming ability, and can prevent the hair from being damaged by permanent waving.

## BEST MODE FOR CARRYING OUT THE INVENTION

Examples of the carbonate useful in the practice of the present invention include sodium carbonate, potassium carbonate, ammonium carbonate, sodium potassium carbonate, lithium carbonate, cesium carbonate, calcium carbonate, magnesium carbonate, etc. Examples of the hydrogencarbonate include sodium hydrogencarbonate, potassium hydrogencarbonate, ammonium hydrogencarbonate, magnesium potassium hydrogencarbonate, lithium hydrogencarbonate, etc. Of these, sodium carbonate, potassium carbonate, ammonium carbonate, sodium potassium carbonate, sodium hydrogencarbonate, potassium hydrogencarbonate, ammonium hydrogencarbonate and magnesium potassium hydrogencarbonate are particularly preferred.

These carbonates and hydrogencarbonates may be used either singly or in any combination thereof, and may preferably be incorporated in a total proportion of 0.5-10 wt.% (hereinafter indicated merely by "%"), particularly 1-5% into the intermediate treatment composition for permanent wave.

The intermediate treatment composition for permanent wave according to the present invention can effectively cleave the mixed disulfides at a pH within a range of from weak acidity to alkalinity. It is also preferable to adjust the pH of the composition to 6-10, particularly 8-10. If the pH is lower than 6, sufficient effects can not be achieved. On the other hand, any pH levels exceeding 10 are not very preferred from the viewpoint of irritation. Examples of pH adjustors used for adjusting the composition to such a pH include weak acids such as citric acid, succinic acid, acetic acid, lactic acid, tartaric acid, glycine and aspartic acid, or salts thereof, alkalis such as sodium hydroxide, potassium hydroxide, ammonia, monoethanolamine, diethanolamine and triethanolamine, basic amino acids such as lysine and arginine, etc.

In the intermediate treatment compositions for permanent wave according to the present invention, optional ingredients which are used routinely as needed, for example, surfactants, solvents, oily substances, moisturizers, colorants, emulsifiers, perfume bases, etc. may be suitably incorporated in addition to the above-described essential ingredients. Besides, a cationic polymer described in Japanese Patent Application Laid-Open No. 92812/1981, amphoteric polymer or silicone such as dimethyl polysiloxane, methylphenyl polysiloxane, cyclic silicone, polyether-modified silicone and amino-modified silicone may also be incorporated to enhance the hair-protective effect.

The intermediate treatment compositions for permanent wave according to the present invention can be formulated by dissolving the essential and optional ingredients in water or an aqueous solvent to form a solution, milky lotion, aerosol or the like.

The thus-obtained intermediate treatment composition for permanent wave according to the present inventions is used by applying or spraying it to the hair between treatments with the first and second compositions in the usual permanent waving process. It is particularly preferable to trea the hair with the first composition which is within a range of from acidity to alkalinity, then with the intermediate treatment composition of this invention, and finally with the second composition.

As the first-package permanent wave composition which is within a range of from acidity to weak alkalinity, may preferably be used a common composition comprising thioglycolic acid, its salt or its ester derivative such as glyceryl monothioglycolate, L-cysteine, N-acetyl-cysteine, thiolactic acid, or the like and adjusted to pH 4-8. As the second-package permanent wave composition, may preferably be used a composition comprising, as a principal component, an oxidizing agent such as a bromate, perborate, hydrogen peroxide or iodine.

## EXAMPLES

The present invention will hereinafter be described in more detail by the following examples. However, it should be borne in mind that this invention is not limited to and by these examples.

Examples 1-3, Comparative Examples 1-2:

In a permanent waving treatment making use of the followi first-and second-package cold wave compositions, the hair was treated with their corresponding intermediate treatment compositions for permanent wave (Examples 1-3, Comparative Example 1) shown in Table 1 between treatments with the first and second compositions. At this time, degree of permanent wave was evaluated by determining degree of waving and diameter of curl. The results are shown in Table 2.

Chemicals used:

| (1) First-package cold wave composition: | |
|---|---|
| Ammonium thioglycolate | 7.0 (%) |
| Water, phosphoric acid (adjusted to pH 6.5 with phosphoric acid) | 93.0 |
| (2) Second-package cold wave composition: | |
| Sodium bromate | 5.0 (%) |
| Water | 95.0 |

Besides, the hair was treated with a first-package cold wave composition having a composition described below and the second composition of the above composition without using any intermediate treatment composition for permanent wave. At this time, degree of permanent wave was also evaluated by determining degree of waving and diameter of curl. The results are shown as Comparative Example 2 in Table 2.

Chemicals used:

| (1) First-package cold wave composition: | |
|---|---|
| Ammonium thioglycolate | 7.0 (%) |
| Ammonium hydrogencarbonate | 4.0 |
| Water, phosphoric acid (adjusted to pH 6.5 with phosphoric acid) | 89.0 |

Table 1

| | Example | | | Comp. Ex. |
|---|---|---|---|---|
| (%) | 1 | 2 | 3 | 1 |
| Ammonium hydrogen-carbonate | 4.0 | | | |
| Sodium carbonate | | 4.0 | | |
| Sodium hydrogen-carbonate | | | 4.0 | |
| Ammonium chloride | | | | 4.0 |
| Deionized water | Balance | Balance | Balance | Balance |
| pH* | 8.0 | 8.0 | 8.0 | 8.0 |

*: Adjusted with phosphoric acid for Examples 1-3, and with sodium hydroxide for Comparative Example 1.

Evaluation methods:

(1) Degree of waving:

Ten Japanese hairs of 15 cm in length, which had been subjected to bleaching and permanent waving each once in accordance with the respective methods known per se in the art, were bundled to wind the resulting bundle around a glass tube (10 mm in diameter). The hair-wound glass tube was immersed for 20 minutes at 30°C in the first composition. The glass tube was immersed for 10 minutes in the intermediate-treatment composition (Examples 1-3, Comparative Example 1), then for 10 minutes in the second composition. After the thus-treated hair bundle was thoroughly washed with water, it was taken out of the glass tube. As a result, the hair bundle was curled in the form of a coil. At this time, the length of a coil of the hair bundle was measured to determine a degree of waving in accordance with the following equation.

Besides, with respect to Comparative Example 2, a hair-wound glass tube was immersed for 30 minutes at 30°C in the first composition and then for 10 minutes directly in the second composition without conducting any intermediate treatment. A degree of waving was then determined in the same manner as described above.

$$\text{Degree of waving} = \frac{X - Y}{X} \times 100$$

X: The whole length of the hair bundle (15 cm)
Y: The length of the coil of the hair bundle(cm).

(2) Diameter of curl:

An inner diameter of the coil of the hair bundle, which had been obtained in the test (1), was measured, and termed "diameter of curl (mm)".

Results:

Table 2

|  | Degree of waving (%) | Diameter of curl (mm) |
|---|---|---|
| Ex. 1 | 40.0 | 19 |
| Ex. 2 | 41.0 | 20 |
| Ex. 3 | 41.5 | 20 |
| Comp. Ex. 1 | 21.5 | 23 |
| Comp. Ex. 2 | 38.0 | 26 |

It is understood from the results of Table 2 that when the intermediate treatment compositions for permanent wave according to the present invention is used, the degree of permanent wave becomes good.

Example 4:

Using the same intermediate treatment composition for permanent wave as used in Example 3, permanent waving was conducted in the same manner as in Examples 1-3 and Comparative Example 1. At this time, changes in modulus of elasticity of the hair after the treatments with the first composition, the intermediate treatment composition for permanent wave and the second composition were determined. The results are shown in Table 3. Incidentally, the numerical values in Table 3 are numerical values calculated regarding the modulus of elasticity of the hair before the first treatment as 100.

Table 3

| Before treatment | After treatment with first composition | After application of intermediate treatment composition for permanent wave | After treatment with second composition |
|---|---|---|---|
| 100 | 51 | 78 | 100 |

It is understood from the results of Table 3 that the use of the intermediate treatment composition for permanent wave according to the present invention does not cause the reduction of modulus of elasticity of the hair.

Example 5:

The hair was treated with an intermediate treatment composition for permanent wave of a composition described below between treatments with the same first and second compositions as used in Example 1. As a result, it was found that degree of permanent wave is good as with Examples 1-3, and damage to the hair is also prevented.

| (Composition) | |
|---|---|
| | (%) |
| Ammonium hydrogencarbonate | 4.0 |
| Propylene glycol | 2.0 |
| Stearyltrimethylammonium chloride (28%) | 1.0 |
| Polyoxyethylene lauryl ether (23 E.O.) | 0.5 |
| Perfume base | 0.05 |
| Amino-modified silicone* | 1.0 |
| Aqueous ammonia (28%) | Proper amount** |
| Deionized water | Balance |
| | 100.0 |

\*: Toray Silicone SM8702C (Toray Industries, Inc.)

\*\*: Amount to pH 8.0.

INDUSTRIAL APPLICABILITY

When the hair is treated with the intermediate treatment composition for permanent wave according to the present invention between treatments with the first and second compositions, mixed disulfides formed in the hair by the treatment with the first composition can be cleaved. Therefore, the wave-forming rate can be enhanced, and damage to the hair can also be prevented.

**Claims**

1. Use of a composition comprising at least one carbonate and/or at least one hydrogencarbonate for cleaving mixed disulfides formed in the hair by a treatment with a reducing agent composition.

2. Use as defined in Claim 1, wherein said at least one carbonate and/or hydrogencarbonate is contained in a proportion of 0.5-10 wt.%.

3. Use as defined in Claim 1 or 2, wherein pH is 6-10.

4. A permanent-waving process, which comprises treating the hair with a first-package permanent wave composition, comprising a reducing agent, with an intermediate treatment composition for permanent wave including at least one carbonate and/or at least one hydrogencarbonate and then with a second-package permanent wave composition comprising an oxidizing agent.

5. The permanent-waving process according to Claim 4, wherein the intermediate treatment composition for permanent wave comprises at least one carbonate and/or at least one hydrogencarbonate in a proportion of 0.5-10 wt.%.

6. The permanent-waving process according to Claim 4 or 5, wherein the pH of the intermediate treatment composition for permanent wave is 6-10.

**Patentansprüche**

1. Verwendung einer Zusammensetzung, umfassend zumindest ein Carbonat und/oder zumindest ein Hydrogencarbonat zum Spalten von gemischten Disulfiden, die im Haar durch Behandlung mit einer Reduktionsmittelzusammensetzung gebildet sind.

2. Verwendung wie in Anspruch 1 definiert, worin das zumindest eine Carbonat und/oder Hydrogencarbonat in einem Anteil von 0,5 bis 10 Gew.-% enthalten sind.

3. Verwendung nach Anspruch 1 oder 2, worin der pH 6 bis 10 ist.

**4.** Dauerwellverfahren, umfassend das Behandeln des Haars mit einer ersten Packung für eine Dauerwellzusammensetzung, umfassend ein Reduktionsmittel, mit einer Zwischenbehandlungszusammensetzung für Dauerwellen, umfassend zumindest ein Carbonat und/oder zumindest ein Hydrogencarbonat, und dann mit einer zweiten Packung für eine Dauerwellzusammensetzung, umfassend ein Oxidationsmittel.

**5.** Dauerwellverfahren nach Anspruch 4, worin die Zwischenbehandlungszusammensetzung für Dauerwellen zumindest ein Carbonat und/oder zumindest ein Hydrogencarbonat in einem Anteil von 0,5 bis 10 Gew.-% umfaßt.

**6.** Dauerwellverfahren nach Anspruch 4 oder 5, worin der pH der Zwischenbehandlungszusammensetzung für Dauerwellen 6 bis 10 ist.

**Revendications**

**1.** Utilisation d'une composition comprenant au moins un carbonate et/ou au moins un hydrogénocarbonate pour cliver des disulfures mixtes formés dans les cheveux par un traitement avec une composition d'un agent réducteur.

**2.** Utilisation telle que définie dans la revendication 1, dans laquelle ledit au moins un carbonate et/ou hydrogénocarbonate est présent en une proportion de 0,5 à 10% en poids.

**3.** Utilisation telle que définie dans la revendication 1 ou 2, dans laquelle le pH est de 6 à 10.

**4.** Procédé d'ondulation permanente, qui comprend le traitement des cheveux avec une composition pour permanente dans un premier emballage comprenant un agent réducteur, avec une composition de traitement intermédiaire pour permanente comprenant au moins un carbonate et/ou au moins un hydrogénocarbonate et ensuite avec une composition pour permanente dans un second emballage comprenant un agent oxydant.

**5.** Procédé d'ondulation permanente selon la revendication 4, dans lequel la composition de traitement intermédiaire pour permanente comprend au moins un carbonate et/ou au moins un hydrogénocarbonate en une proportion de 0,5 à 10% en poids.

**6.** Procédé d'ondulation permanente selon la revendication 4 ou 5, dans lequel le pH de la composition de traitement intermédiaire pour permanente est de 6 à 10.